(19) Europäisches Patentamt
European Patent Office
Office européen des brevets

(11) Veröffentlichungsnummer: **0 319 942 A2**

## (12) EUROPÄISCHE PATENTANMELDUNG

(21) Anmeldenummer: **88120419.2**

(22) Anmeldetag: **07.12.88**

(51) Int. Cl.⁴: **C07C 103/46 , C07C 101/12 , C07C 143/74 , C10M 133/06 , C10M 173/02 , C10M 169/04 , C09D 7/06 , //E21B43/22, B01F17/00,(C10M173/02, 133:06),(C10M169/04,107:34, 133:06),C10N30:06,C10N40:00, C10N40:20,C10N40:22**

(30) Priorität: **07.12.87 CH 4754/87**

(43) Veröffentlichungstag der Anmeldung: **14.06.89 Patentblatt 89/24**

(84) Benannte Vertragsstaaten: **AT BE DE ES FR GB IT NL SE**

(71) Anmelder: **Bereuter, Heinz, Dipl.-Ing.**
**An der Steinlache 10**
**A-6971 Hard(AT)**

(72) Erfinder: **Bereuter, Heinz**
**An der Steinlache 10**
**A-6971 Hard(AT)**
Erfinder: **Bereuter, Wolfgang**
**An der Steinlache 10**
**A-6971 Hard(AT)**
Erfinder: **Bereuter, Thomas**
**An der Steinlache 10**
**A-6971 Hard(AT)**

(74) Vertreter: **Büchel, Kurt F., Dr.**
**Patentanwalt Dr. Kurt F. Büchel Bergstrasse 297**
**FL-9495 Triesen(LI)**

# (54) Neue Salze und Amine von wenigstens teilweise perfluorierten Amino- und/oder Amidocarbonsäuren sowie deren Verwendung.

(57) Die Salze und Amine haben eine Struktur folgender Formel:

R1-N-(CH2)m-N-R2
CH-R4 R3
(CH2)n
COOH

EP 0 319 942 A2

wobei

R1 einen perfluorierten Alkanoyl-, Alkylsulfonyl-, Alkylacetyl- oder Alkyläthylenrest mit jeweils 6 bis 16 Kohlenstoffatomen;

R2 ein Wasserstoffatom, oder den Rest R1, oder das Radikal - $\underset{R4}{CH}$ $-(CH_2)_n-COOH$;

R3 das Radikal - $\underset{R4}{CH}$ $-(CH_2)_n-COOH$

oder

$-[(CH_2)_m-N]_z-$ $\underset{R4}{CH}$ $-(CH_2)_n-COOH$;

R4 einen Wasserstoff; oder einen Alkylrest mit 1 bis 3 Kohlenstoffatomen;

m eine ganze Zahl von 2 bis 6;

n eine ganze Zahl von 0 bis 2; und

z eine ganze Zahl von 1 bis 6 bedeuten.

und werden für die Herstellung von Kühlschneidmitteln, Bohrhilfsmitteln, Hydraulik-und Schmierflüssigkeiten, sowie als Netz- oder Dispergierhilfsmittel oder Emulgatoren verwendet.

## Neue Salze und Amine von wenigstens teilweise perfluorierten Amino- und/oder Amidocarbonsäuren, sowie deren Verwendung

Es ist bekannt, dass langkettige Perfluoralkylcarbonsäuren, Perfluoralkylsulfonsäuren und Perfluoralkanoylamidocarbonsäuren, wie sie z.B. in der DE-AS 1 242 626 genannt werden, in Form ihrer Salze bereits in 0,05%-iger, wässriger Lösung so gute Schmierwirkung zeigen, wie sie sonst nur von hochlegierten Kühl- und Schmierölen erreicht werden. Die derartigen Salze sind aber so starke Schäumer, dass sie als EP-Zusätze ungeeignet sind.

Ueberraschend wurde nun gefunden, dass Alkali- und Aminsalze von Perfluoralkanoylamidocarbonsäuren, Perfluoralkylsulfonamidocarbonsäuren, Perfluoralkylacetylamidocarbonsäuren und Perfluoralkyläthylenaminocarbonsäuren der allgemeinen Formel

```
R1-N-(CH2)m-N-R2
   |        |
   CH-R4    R3
   |
  (CH2)n
   |
   COOH
```

trotz des verminderten Fluorgehaltes im Molekül bei gleicher Konzentration in g/l den gleich guten Schmiereffekt aufweisen wie die Alkali- und Aminsalze der zugrunde liegenden Perfluoralkylverbindungen. Obwohl die Grenzflächenaktivität noch weiter gesteigert wird, zeigen derartige Verbindungen eine weit geringere Schaumwirkung, so dass sie als wasserlösliche EP-Zusätze geeignet erscheinen.

In der Formel bedeuten:

R1 einen Perfluoralkanoylrest mit 6-16 Kohlenstoffatomen oder einen Perfluoralkylsulfonylrest mit 6-16 Kohlenstoffatomen oder einen Perfluoralkylacetylrest mit 6-16 Kohlenstoffatomen oder einen Perfluoralkyläthylenrest mit 6-16 Kohlenstoffatomen;

R2 den Rest R1 oder das Radikal $-\underset{R4}{\underset{|}{C}}H-(CH2)n-COOH$ oder ein Wasserstoffatom;

R3 das Radikal $-\underset{R4}{\underset{|}{C}}H-(CH2)n-COOH$ oder $-[(CH2)m-N]z-\underset{R4}{\underset{|}{C}}H-(CH2)n-COOH$;

R4 einen Alkylrest mit 1 bis 3 Kohlenstoffatomen oder ein Wasserstoffatom;

m eine ganze Zahl von 2 bis 6;

n eine ganze Zahl von 0 bis 2; und

z eine ganze Zahl von 1 bis 6.

Es ist für die spanabhebende Metallfertigung von besonderer Bedeutung, ölfreie, schwer brennbare Hydraulik- und Schmierflüssigkeiten zur Verfügung zu haben, deren Leckage die wasserlöslichen und wassermischbaren Kühl-Schmiermittel in ihrer Funktion nicht beeinträchtigt. Ebenso wichtig ist es, die diversen Kühl-, Schneid- und Schmieröle durch wasserlösliche Substanzen, wie zum Beispiel ein erfindungsgemässes Salz oder Amin, oder durch eine Mischung von mehreren der erfindungsgemässen Salze oder Amine mit geeigneten Korrosionsinhibitoren, wie sie z.B. im Schweizer Patent Nr. 614735 genannt sind, und geeigneten Verdickungsmitteln zu ersetzen.

Durch Mischung eines oder mehrerer der erfindungsgemässen Salze oder Amine mit geeigneten Polyglykolen ist es möglich, Schmierflüssigkeiten für Motoren und Getriebe von hervorragender Schmierwirkung und extremer Temperaturbeständigkeit herzustellen.

Erfindungsgemäss seien folgende chemische Substanzen beispielhaft angeführt:

1. N-perfluoroctylacetyl-äthylendiamin-N,N′-diessigsäure
2. N,N′-diperfluoroctylacetyl-äthylendiamin-N,N′-diessigsäure
3. N-perfluoroctylacetyl-äthylendiamin-N,N′,N′-triessigsäure
4. N-perfluorhexylacetyl-äthylendiamin-N,N′-diessigsäure
5. N,N′-diperfluorhexylacetyl-äthylendiamin-N,N′-diessigsäure
6. N-perfluorhexylacetyl-äthylendiamin-N,N′,N′-triessigsäure
7. N-perfluoroctanoyl-äthylendiamin-N,N′-diessigsäure

8. N,N′-diperfluoroctanoyl-äthylendiamin-N,N′-diessigsäure
9. N-perfluoroctanoyl-äthylendiamin-N,N′,N′-triessigsäure
10. N-perfluorhexanoyl-äthylendiamin-N,N′-diessigsäure
11. N,N′-diperfluorhexanoyl-äthylendiamin-N,N′-diessigsäure
12. N-perfluorhexanoyl-äthylendiamin-N,N′,N -triessigsäure
13. N-perfluordecanoyl-äthylendiamin-N,N′-diessigsäure
14. N,N′-diperfluordecanoyl-äthylendiamin-N,N′-diessigsäure
15. N-perfluordecanoyl-äthylendiamin-N,N′,N′-triessigsäure
16. N,N′-diperfluordodecanoyl-äthylendiamin-N,N′-diessigsäure
17. N-perfluordodecanoyl-äthylendiamin-N,N′,N′-triessigsäure
18. N,N′-diperfluor-9-isodecanoyl-äthylendiamin-N,N′-diessigsäure
19. N-perfluoroctylsulfonyl-äthylendiamin-N,N′-diessigsäure
20. N,N′-diperfluoroctylsulfonyl-äthylendiamin-N,N′-diessigsäure
21. N-perfluoroctylsulfonyl-äthylendiamin-N,N′,N′-triessigsäure
22. N,N′-diperfluoroctyläthylen-äthylendiamin-N,N′-diessigsäure
23. N-perfluoroctylacetyl-äthylendiamin-N,N′,N′-tripropionsäure
24. N,N′-diperfluoroctylacetyl-äthylendiamin-N,N′-dipropionsäure
25. N-perfluoroctylacetyl-äthylendiamin-N,N′-dipropionsäure
26. N,N′-diperfluoroctanoyl-äthylendiamin-N,N′-dipropionsäure
27. N-perfluoroctanoyl-äthylendiamin-N,N′,N′-tripropionsäure
28. N,N′-diperfluorisodecanoyl-äthylendiamin-N,N′-dibuttersäure
29. N-perfluorisodecanoyl-äthylendiamin-N,N′,N′-tributtersäure
30. N,N″-diperfluoroctanoyl-diäthylentriamin-N,N′,N″-triessigsäure
31. N-perfluoroctanoyl-diäthylentriamin-N,N′,N″-triessigsäure
32. N,N′-diperfluoroctylacetyl-propylendiamin-N,N′-diessigsäure
33. N-perfluoroctylacetyl-propylendiamin-N,N′,N′-triessigsäure

Die erfindungsgemässen Säuren können mit den entsprechenden Basen, wie beispielsweise Alkalien oder Aminen, vorzugsweise Alkanolaminen wie Mono-, Di- oder Trialkanolaminen im äquivalenten Verhältnis oder auch im Ueberschuss umgesetzt und in Mischung mit bekannten Korrosionsinhibitoren in wässriger Lösung angewendet werden.

Ueberraschend wurde gefunden, dass Mischungen der angeführten chemischen Verbindungen bei gleich gutem Schmiereffekt noch geringere Schaumwirkung zeigen als die reinen Komponenten.

## Schaumprüfung

Die vergleichenden Schaumprüfungen wurden nach dem Lochscheiben-Schlagverfahren nach DIN 53902, Teil 1 durchgeführt. In einem 1-Liter Messzylinder werden 200 ml der zu prüfenden Lösung, die mit destilliertem Wasser hergestellt wurde, eingefüllt. Durch diese Lösung von 20 Grad C wird eine an einem Stiel befestigte Lochscheibe von 55 mm Durchmesser mit 40 Bohrungen von 4,5 mm Durchmesser in 30 sec. 30 mal mechanisch geschlagen. Die Hubhöhe der Lochplatte beträgt 350 mm. Das Schaumvolumen der Probenlösung wird 30 sec. nach dem Schlagen gemessen.

| Nummer d. Verbindung als Triäthanolaminsalz | Konzentration in Prozenten | Schaumhöhe nach 30 sec. |
|---|---|---|
| 1 | 0,05% | 0 mm |
| 2 | 0,05% | 17 mm |
| 3 | 0,05% | 0 mm |
| 2 und 3 (50 : 50) | 0,05% | 0 mm |
| 23 | 0,05% | 0 mm |
| 24 | 0,05% | 20 mm |
| 25 | 0,05% | 0 mm |
| 23 und 24 (50 : 50) | 0,05% | 0 mm |
| 32 | 0,05% | 20 mm |
| 33 | 0,05% | 0 mm |
| 32 und 33 (50 : 50) | 0,05% | 0 mm |

Zum Vergleich:

| | | |
|---|---|---|
| Perfluoroctylessigsäure-Triäthanolaminsalz | 0,05% | 77 mm |
| Perfluoroctylacetyl-amido-essigsäure-Triäthanolaminsalz | 0,05% | 63 mm |
| 5 | 0,05% | 0 mm |
| 6 | 0.05% | 0 mm |

Zum Vergleich:

| | | |
|---|---|---|
| Perfluorhexylessigsäure als Triäthanolaminsalz | 0,05% | 60 mm |

| Nummer d. Verbindung als Triäthanolaminsalz | Konzentration in Prozent | Schaumhöhe nach 30 sec. |
|---|---|---|
| 7 | 0,05% | 0 mm |
| 8 | 0,05% | 23 mm |
| 9 | 0,05% | 0 mm |
| 30 | 0,05% | 3 mm |
| 31 | 0,05% | 0 mm |
| 8 und 9 (40 : 60) | 0,05% | 0 mm |
| 30 und 31 (50 : 50) | 0,05% | 0 mm |

Zum Vergleich:

| | | |
|---|---|---|
| Perfluoroctylessigsäure-Triäthanolaminsalz | 0,05% | 105 mm |
| Perfluoroctanoyl-amidoessigsäure-Triäthanolaminsalz | 0,05% | 85 mm |

| Nummer d. Verbindung als Triäthanolaminsalz | Konzentration in Prozent | Schaumhöhe nach 30 sec. |
|---|---|---|
| 13 | 0,05% | 0 mm |
| 14 | 0,05% | 25 mm |
| 15 | 0,05% | 0 mm |
| 14 und 15 (40 : 60) | 0,05% | 0 mm |
| <u>Zum Vergleich:</u> | | |
| Perfluordecanoylessigsäure-Triäthanolaminsalz | 0,05% | 112 mm |
| Perfluordecanoyl-amido-essigsäure-Triäthanolaminsalz | 0,05% | 90 mm |
| 16 | 0,05% | 110 mm |
| 17 | 0,05% | 0 mm |
| 16 und 17 (40 : 60) | 0,05% | 20 mm |
| 18 | 0,05% | 23 mm |
| 20 | 0,05% | 25 mm |
| 21 | 0,05% | 0 mm |
| 20 und 21 (50 : 50) | 0,05% | 3 mm |
| 28 | 0,05% | 25 mm |
| 29 | 0,05% | 0 mm |
| 28 und 29 (40 : 60) | 0,05% | 0 mm |

<u>Schmierprüfungen:</u>

Die vergleichenden Schmierprüfungen wurden auf der "Reibverschleisswaage nach Reichert" durchgeführt. Dabei wird über ein Doppelhebelsystem eine fest eingespannte, genormte Prüfrolle an einen umlaufenden, genormten Schleifring angepresst, der in seinem unteren Drittel in die zu prüfende Flüssigkeit taucht, deren Druckaufnahmevermögen beurteilt werden soll. Die Drehzahl des Schleifringes ist so bemessen, dass stets genügend Flüssigkeit in die Berührungs- (Reibverschleiss-) stelle zwischen Prüfrolle und Schleifring eintreten kann. Die scharf abgegrenzte Abriebellipse wird nach 100 Schleifmetern auf der Prüfrolle gemessen (in mm). Bei allen Versuchen wurde mit einem Belastungsgewicht von 1500 g, das entspricht einem Anpressdruck von 30 kg, gefahren. Es wurden jeweils ein Vor-und Nachlauf in destilliertem Wasser und 3 Probeläufe mit der Prüflösung durchgeführt.

Nummer der Verbindung als Triäthanolaminsalz

(V=Vorlauf, N=Nachlauf, beide mit destilliertem Wasser)

-- 1 --

0,05% in dest. Wasser

| | Abrieb | mm2 |
|---|---|---|
| V | 8.6 x 5.3 | 35.8 |
| | 6.4 x 3.9 | 19.6 |
| | 4.3 x 2.7 | 9.1 |
| | 4.2 x 2.6 | 8.9 |
| N | 8.5 x 5.2 | 34.7 |

-- 2 --

0,05% in dest. Wasser

| | Abrieb | mm2 |
|---|---|---|
| V | 8.6 x 5.2 | 35.1 |
| | 6.2 x 3.7 | 18.3 |
| | 2.3 x 1.4 | 2.5 |
| | 2.4 x 1.5 | 2.8 |
| N | 8.6 x 5.1 | 34.4 |

-- 2 --

0,025% in dest. Wasser

| | Abrieb | mm2 |
|---|---|---|
| V | 8.6 x 5.3 | 35.8 |
| | 6.9 x 4.3 | 23.3 |
| | 6.8 x 4.2 | 22.4 |
| | 6.1 x 3.4 | 16.3 |
| N | 8.7 x 5.1 | 34.8 |

Nummer der Verbindung als Triäthanolaminsalz

(V=Vorlauf, N=Nachlauf, beide mit destilliertem Wasser)

-- 2 --

0,2% in dest. Wasser

| | Abrieb | mm2 |
|---|---|---|
| V | 8.5 x 5.2 | 34.7 |
| | 3.3 x 2.2 | 5.7 |
| | 2.1 x 1.4 | 2.3 |
| | 2.0 x 1.3 | 2.0 |
| N | 8.3 x 5.0 | 32.6 |

-- 2 --

0,1% in dest. Wasser

| | Abrieb | mm2 |
|---|---|---|
| V | 8.4 x 5.2 | 34.3 |
| | 3.4 x 2.1 | 5.6 |
| | 2.2 x 1.5 | 2.6 |
| | 2.1 x 1.4 | 2.3 |
| N | 8.3 x 4.9 | 31.9 |

-- 3 --

0,05% in dest. Wasser

| | Abrieb | mm2 |
|---|---|---|
| V | 8.7 x 5.3 | 36.2 |
| | 6.3 x 3.8 | 18.8 |
| | 4.4 x 2.7 | 9.3 |
| | 4.4 x 2.8 | 9.7 |
| N | 8.7 x 5.1 | 34.8 |

-- 2 und 3 --, 50:50

0,05% in dest. Wasser

| | Abrieb | mm2 |
|---|---|---|
| V | 8.6 x 5.0 | 33.8 |
| | 5.5 x 3.4 | 14.7 |
| | 2.3 x 1.4 | 2.5 |
| | 2.3 x 1.3 | 2.3 |
| N | 8.6 x 5.1 | 34.4 |

-- 23 --

0,05% in dest. Wasser

| | Abrieb | mm2 |
|---|---|---|
| V | 8.6 x 5.3 | 35.8 |
| | 6.2 x 3.9 | 18.9 |
| | 4.3 x 2.7 | 9.1 |
| | 4.4 x 2.7 | 9.3 |
| N | 8.5 x 5.1 | 34.0 |

-- 24 --

0,05% in dest. Wasser

| | Abrieb | mm2 |
|---|---|---|
| V | 8.6 x 5.2 | 35.1 |
| | 5.9 x 3.5 | 16.2 |
| | 2.2 x 1.5 | 2.6 |
| | 2.2 x 1.4 | 2.4 |
| N | 8.6 x 5.0 | 33.8 |

Nummer der Verbindung als Triäthanolaminsalz (V=Vorlauf, N=Nachlauf, beide mit destilliertem Wasser)

-- 32 --

0,05% in dest. Wasser

| | Abrieb | mm2 |
|---|---|---|
| V | 8.5 x 5.2 | 34.7 |
| | 6.3 x 4.1 | 20.3 |
| | 2.1 x 1.3 | 2.1 |
| | 2.2 x 1.4 | 2.4 |
| N | 8.5 x 5.3 | 35.4 |

-- 33 --

0,05% in dest. Wasser

| | Abrieb | mm2 |
|---|---|---|
| V | 8.6 x 5.2 | 35.1 |
| | 6.4 x 4.2 | 21.1 |
| | 4.4 x 2.8 | 9.7 |
| | 4.3 x 2.9 | 9.8 |
| N | 8.6 x 5.2 | 35.1 |

-- 23 und 24 --,50:50

0.05% in dest. Wasser

| | Abrieb | mm2 |
|---|---|---|
| V | 8.6 x 5.3 | 35.8 |
| | 6.1 x 3.7 | 17.7 |
| | 2.4 x 1.5 | 2.8 |
| | 2.3 x 1.5 | 2.7 |
| N | 8.5 x 5.2 | 35.7 |

-- 32 und 33 --,50:50

0,05% in dest. Wasser

| | Abrieb | mm2 |
|---|---|---|
| V | 8.7 x 5.2 | 35.5 |
| | 6.3 x 4.1 | 20.3 |
| | 2.5 x 1.4 | 2.7 |
| | 2.4 x 1.5 | 2.8 |
| N | 8.6 x 5.1 | 34.4 |

<u>Zum Vergleich:</u>

0,05% Perfluoroctylessigsäure als Triäthanolaminsalz in destilliertem Wasser

| | Abrieb | mm2 |
|---|---|---|
| V | 8.5 x 5.2 | 34.7 |
| | 6.4 x 3.9 | 19.6 |
| | 2.2 x 1.4 | 2.4 |
| | 2.3 x 1.4 | 2.5 |
| N | 8.5 x 4.9 | 32.7 |

0,025% Perfluoroctylessigsäure als Triäthanolaminsalz in in destilliertem Wasser

| | Abrieb | mm2 |
|---|---|---|
| V | 8.6 x 5.3 | 35.8 |
| | 6.6 x 4.0 | 20.8 |
| | 6.4 x 3.8 | 19.1 |
| | 5.7 x 3.1 | 17.4 |
| N | 8.7 x 5.1 | 34.8 |

0,05% Perfluoroctylacetyl-amidoessigsäure(DAS 1242626) als Triäthanolaminsalz in destilliertem Wasser

| | Abrieb | mm2 |
|---|---|---|
| V | 8.7 x 5.1 | 34.8 |
| | 6.2 x 3.7 | 18.0 |
| | 2.1 x 1.3 | 2.1 |
| | 2.0 x 1.4 | 2.2 |
| N | 8,8 x 5.0 | 33.4 |

0,025% Perfluoroctylacetyl-amidoessigsäure(DAS 1242626) als Triäthanolaminsälz in destilliertem Wasser

| | Abrieb | mm2 |
|---|---|---|
| V | 8.6 x 5.2 | 35.1 |
| | 6.2 x 3.5 | 17.1 |
| | 6.4 x 3.8 | 19.1 |
| | 6.0 x 4.0 | 18.7 |
| N | 8.8 x 5.0 | 33.4 |

Nummer der Verbindung als Triäthanolaminsalz

(V=Vorlauf, N=Nachlauf, beide mit destilliertem Wasser)

-- 5 --

0,05% in dest. Wasser

| | Abrieb | mm2 |
|---|---|---|
| V | 8.7 x 5.1 | 34.8 |
| | 7.0 x 4.3 | 23.7 |
| | 6.7 x 4.1 | 21.6 |
| | 6.7 x 4.2 | 22.1 |
| N | 8.6 x 5.2 | 35.1 |

-- 14 --

0,05 % in dest. Wasser

| | Abrieb | mm2 |
|---|---|---|
| V | 8.7 x 5.2 | 35.5 |
| | 5.1 x 3.2 | 12.8 |
| | 5.8 x 3.5 | 15.9 |
| | 5.0 x 3.0 | 11.8 |
| N | 8.6 x 5.2 | 35.1 |

-- 20 --

0,05% in dest. Wasser

| | Abrieb | mm2 |
|---|---|---|
| V | 8.5 x 5.2 | 34.7 |
| | 4.4 x 2.9 | 10.0 |
| | 2.0 x 1.3 | 2.0 |
| | 2.1 x 1.4 | 2.3 |
| N | 8.4 x 5.1 | 33.6 |

-- 26 --

0,05 % in dest. Wasser

| | Abrieb | mm2 |
|---|---|---|
| V | 8.6 x 5.2 | 35.1 |
| | 6.0 x 4.0 | 18.7 |
| | 2.2 x 1.5 | 2.6 |
| | 2.1 x 1.4 | 2.3 |
| N | 8.4 x 5.2 | 34.3 |

Die erfindungsgemässen Salze und können nun, wie be reits angedeutet, nicht nur für die Metallbearbeitung, sondern besonders vorteilhaft auch in anderen Anwendungsbereichen eingesetzt werden, in denen es auf gute Schmierwirkung, geringe Schaumbildung und gute Netzeigenschaften ankommt. Als besonders interessant haben sie sich in denjenigen Gebieten und denjenigen Konzentrationen gezeigt, wie sie in den Ansprüchen 2 bis 8 beschrieben sind.

Dazu gehört unter anderem auch die Gesteinsbohrung, bei der mit Hilfe der erfindungsgemässen Salze oder Amine der Schneidwiderstand reduziert wird, wodurch die Temperatur in Schneidennähe abnimmt und der Vorschub des Bohrmeissels gesteigert werden kann.

## Ansprüche

1. Salz oder Amin einer wenigstens teilweise perfluorierten Amido- und/oder Aminocarbonsäure, die eine Struktur nach folgender Formel besitzt:

```
R1-N-(CH2)m-N-R2
   |         |
   CH-R4     R3
   |
  (CH2)n
   |
   COOH
```

wobei

R1 einen perfluorierten Alkanoyl-, Alkylsulfonyl-, Alkylacetyl- oder Alkyläthylenrest mit jeweils 6 bis 16 Kohlenstoffatomen;

R2 ein Wasserstoffatom, oder den Rest R1, oder das Radikal -CH(R4)-(CH2)n-COOH;

R3 das Radikal -CH(R4)-(CH2)n-COOH

oder

-[(CH2)m-N]z-CH(R4)-(CH2)n-COOH;

R4 einen Wasserstoff; oder einen Alkylrest mit 1 bis 3 Kohlenstoffatomen;

m eine ganze Zahl von 2 bis 6;

n eine ganze Zahl von 0 bis 2; und

z eine ganze Zahl von 1 bis 6 bedeuten.

2. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Kühlschneidmittel für die Metallbearbeitung.

3. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Bohrhilfsmittel für Gesteinsbohrungen, insbesondere in der Erdöl- und Erdgas-Exploration.

4. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Hilfsmittel für die Sekundär- und/oder Tertiärförderung von Erdöl.

5. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Hydraulikflüssigkeit.

6. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Schmierflüssigkeit zur Schmierung von Maschinen, Maschinenteilen, Motoren und/oder Getrieben.

7. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Netz- oder Dispergierhilfsmittel oder Emulgator.

8. Verwendung eines oder mehrerer verschiedener Salze oder Amine nach Anspruch 1 in wässriger Lösung in einer Konzentration von 0.001 bis 5 Gewichtsprozent, vorzugsweise von 0.01 bis 1 Gewichtsprozent, insbesondere von 0.05 bis 0.1 Gewichtsprozent als Verlaufhilfsmittel für Lacke und Anstriche.